# EUROPÄISCHE PATENTANMELDUNG

(11) **EP 3 495 346 A1**
(43) Veröffentlichungstag der Anmeldung: **12.06.2019**
(21) Anmeldenummer: 17205981.8
(22) Anmeldetag: 07.12.2017
(51) Int. Cl.: C07C 319/20, C07C 323/58, A23P 10/20

(54) **VERFAHREN ZUR HERSTELLUNG VON CALCIUMMETHIONINAT**

(71) Anmelder: Evonik Degussa GmbH, 45128 Essen (DE)
(72) Erfinder: FISCHER, Daniel, Midlothian, VA 23113 (US)

(57) **Zusammenfassung**

Gegenstand der Erfindung ist ein Verfahren zur Herstellung von Calciummethioninat (Ca(Met)₂) durch zweistufige Verseifung von 5-(2-Methylmercaptoethyl)-hydantoin (Methionin-Hydantoin) mit Ammoniak in einer ersten Stufe und Ca(OH)₂ in einer zweiten Stufe, sowie die Isolierung des Produktes durch Sprühgranulation der Reaktionsmischung.

## Beschreibung

Gegenstand der Erfindung ist ein Verfahren zur Herstellung von Calciummethioninat (Ca(Met)₂) durch zweistufige Verseifung von 5-(2-Methylmercaptoethyl)-hydantoin (Methionin-Hydantoin) mit Ammoniak in einer ersten Stufe und Ca(OH)₂ in einer zweiten Stufe, sowie die Isolierung des Produktes durch Sprühgranulation der Reaktionsmischung.

### Stand der Technik:

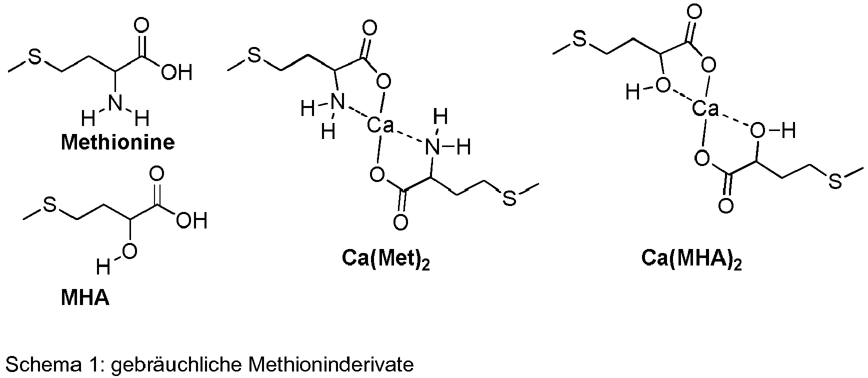

Methionin sowie wäßrige Lösungen von Methioninsalzen, insbesondere von Natriummethioninat (DE 31 05 009 C), aber auch Ersatzstoffe wie das Methionin-Hydroxyanaloge (MHA) oder sein Calciumsalz (Schema 1) werden weltweit als Futtermittelzusatz für die Aufzucht von Geflügel, Schweinen und anderen Nutztieren verwendet und kommen hauptsächlich der Produktion von tierischem Eiweiß zugute. Gerade im Hinblick auf die steigende Weltbevölkerung und die zunehmenden Ernährungsprobleme kommt dem Methionin als einer der erstlimitierenden Aminosäuren im tierischen Wachstumsprozeß und seinen verschiedenen Formen und damit auch deren kostengünstiger Herstellung eine besondere Bedeutung zu. Je nach Erfordernissen werden Feststoff oder Flüssigformen bevorzugt eingesetzt.

In diesem Zusammenhang wurde auch das Calciummethioninat (Schema 1) beschrieben (EP 130281 A1), das als eine weitere feste Form von Methionin Verwendung finden kann. In EP 130281 A1 wurde z.B. hervorgehoben, dass Calciummethioninat die Methioninversorgung von Wiederkäuern im Hinblick auf Gewichtszunahme und Milchleistung verbessert.

Die Patentanmeldung WO 2007034065 (Adisseo) beschreibt die Verseifung von Methionin-Hydantoin mit Ammoniak zum Ammoniummethioninat, wobei anschließend Methionin durch Abstrippen von Ammoniak freigesetzt wird.

DE 19707380 A1 (Degussa) offenbart ein Formgebungsverfahren zur Überführung von Natrium- und Kaliummethioninatlösungen in die entsprechenden Granulate dieser Methioninsalze jedoch kein Verfahren zur Herstellung von Calciummethioninat oder einer diesbezüglichen Feststoffformulierung.

US 3,617,297 (Sumitomo) beschreibt ein Verfahren zur Herstellung von Calciummethioninat direkt durch Verseifung von Methionin-Hydantoin mit einem Überschuss an Ca(OH)₂. Calciummethioninat entsteht dabei jedoch nur als primäres Produkt, welches anschließend mit Phosphorsäure behandelt wird, um das dabei entstehende Methionin und Calciumphosphat auszufällen, während CO₂ entweichen kann. Will man jedoch nur Calciummethioniat durch Verseifung von Methionin-Hydantoin mit Ca(OH)₂ herstellen taucht das Problem auf, dass das im Hydantoin chemisch gebundene CO₂ 1 Equivalent Ca(OH)₂ wegfängt und damit gleichzeitig schwerlösliches CaCO₃ bildet. Eine Abtrennung vom Methionin wäre sehr aufwändig. Wenn man CaCO₃ im Endprodukt belässt, wird dieses entsprechend mit CaCO₃ verdünnt. Es wäre so nur eine maximale Konzentration von 53 Gew-% Methionin, dem eigentlichen Wirkstoff, im Calciummethioniat-Endprodukt möglich.

### Aufgabe:

Es war daher Aufgabe der Erfindung, ein Verfahren zur Herstellung von Calciummethioninat bereitzustellen, das zumindest indirekt ausgehend von den in der industriellen Methionin-Synthese verwendeten Rohstoffen Methylmercaptopropionaldehyd (MMP) und Cyanwasserstoff oder ausgehend von Methylmercaptopropionaldehyd-Cyanhydrin (MMP-CH) direkt zum Calciumsalz des Methionins führt, dergestalt, dass ein möglichst deutlich über 53 Gew-% liegender Methioningehalt im Calciummethioniat-Endprodukt erhalten wird.

### Beschreibung der Erfindung

Diese und weitere Aufgaben werden gelöst durch ein Verfahren zur Herstellung von Calciummethioninat, gekennzeichnet durch folgende Schritte:
a) Hydrolyse von 5-(2-Methylmercaptoethyl)-hydantoin mit Ammoniak und Wasser zu einem Ammoniummethioninat und Kohlendioxid enthaltenden Hydrolysat I ,
b) Erzeugung eines entgeisteten, also von flüchtigen Komponenten befreiten, Hydrolysats I durch wenigstens teilweise Abtrennung von vorhandenem Ammoniak und Kohlendioxid aus dem Hydrolysat I vorzugsweise durch Abdestillieren und/oder Abstrippen,
c) Hydrolyse des entgeisteten Hydrolysats I aus b) mit CaO und/oder Ca(OH)₂ und Wasser zu einem Calciummethioninat und Ammoniak enthaltenden Hydrolysat II,
d) optional Erzeugung eines Calciummethioninat enthaltenden Rohprodukts durch Abtrennung von vorhandenem Ammoniak aus dem Hydrolysat II aus c) vorzugsweise durch Abdestillieren und/oder Abstrippen und
e) Erzeugung von festem Calciummethioninat durch Sprühgranulation des Rohproduktes aus d) oder gegebenenfalls, also wenn Schritt d) weggelassen wird, durch direkte Sprühgranulation des Calciummethioninat und Ammoniak enthaltenden Hydrolysats II aus c).
Die erfindungsgemäße Lösung des Problems mittels einer zweistufigen Verseifung des Methionin-Hydantoins (Schritte a) und c)) hat den Vorteil, dass im Schritt a) durch partielle Hydrolyse mit Ammoniak als Base bereits ein großer Teil des chemisch gebundenen CO₂ in Ammoniumcarbonat/Hydrogencarbonat überführt wird, aus dem anschließend das darin gebundene Ammoniak und CO₂ z.B. durch Abstrippen leicht abgetrennt werden kann (Schritt b)). So wird die unerwünschte Reaktion mit dem anschließend in Schritt c) zugefügten CaO bzw. Ca(OH)₂ zu Calciumcarbonat auf elegante Art weitgehend verhindert. Als Ammoniakquelle kann wässriger Ammoniak in jeder gewünschten Konzentration von ca. 10 bis ca.80 Gew.%, vorzugsweise von 25 bis 50 Gew.% eingesetzt werden. Besonders vorteilhaft ist die Verwendung von ca.33 Gew.% wässrigem Ammoniak. Alternativ kann auch Ammoniak-Gas zusammen mit Wasser im entsprechend gewünschten Verhältnis eingesetzt werden. Die Vervollständigung der Hydantoin-Verseifung mit CaO und/oder Ca(OH)₂ in Schritt c) gelingt in hohen Ausbeuten und führt direkt zu einem Calciummethioninat und Ammoniak enthaltenden Hydrolysat II, aus dem durch Abtrennung von freigesetztem bzw. noch vorhandenem Ammoniak ein Calciummethioninat enthaltendes Rohprodukt erzeugt wird (Schritt e) optional Schritte d) und e), das in der Regel als Suspension anfällt. Die dabei ungelösten Bestandteile umfassen vor allem schwerlösliches Calciumcarbonat, das hier noch zu einem relativ kleinen Anteil gebildet wird, da die Nebenreaktion mit CO₂ nur noch in geringem Maße auftritt, als auch etwas Calciummethioninat je nach eingestellter Konzentration und Temperatur. Der vergleichsweise geringe Anteil an Calciumcarbonat kann im Produkt verbleiben, weil Calciumcarbonat selbst ein zugelassener Futtermittelzusatzstoff ist.
Aus dem Rohprodukt wird in sehr vorteilhafter, weil einfacher Weise durch Sprühgranulation (Schritt e)) das verkaufsfähige Produkt hergestellt. So wird das Entstehen von Mutterlaugen verhindert, die sonst wie beim Standardverfahren zur Methioninherstellung aufwendig aufgearbeitet oder ausgeschleust werden müßten, wobei stets auch noch ein Anteil an Methionin und anderen Wertstoffen (z.B. Met-Met) verlorengeht.

Das Verfahren kann sowohl kontinuierlich als auch diskontinuierlich durchgeführt werden. Insbesondere die kontinuierliche Verfahrensweise ist für eine industrielle Produktion sehr vorteilhaft. 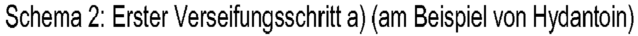 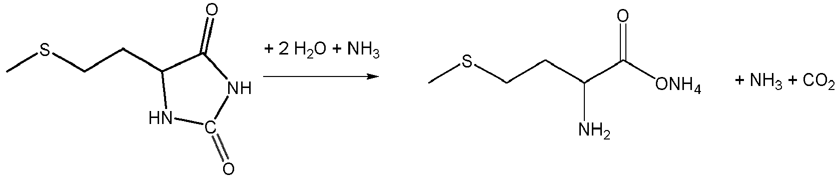 Der Hydrolyse-Schritt a) wird vorteilhaft bei Temperaturen von 140 bis 200°C, vorzugsweise von 155 bis 185°C und besonders bevorzugt von 160 bis 180°C und Drücken von 10 bis 30 barü durchgeführt.
Dabei können im Hydrolyse-Schritt a) von 1 bis 20 Molequivalente (moleq) NH₃, vorzugsweise von 2 bis 10 moleq NH₃ pro moleq Methioninequivalent verwendet werden. Dies gewährleistet hohe Umsätze bei relativ kurzen Verweilzeiten. Unter Methioninequivalent (ME) werden qualitativ hierbei Methionin selbst sowie alle durch Hydrolyse in Methionin(salz) überführbare Substanzen verstanden. Quantitativ betrachtet bezieht der Begriff Methioninequivalent die molare Anzahl von Methionineinheiten mit ein (vgl. Met-Met). Die durch Hydrolyse in Methionin(salz) überführbare Substanzen, in Summe auch als "Hyd" bezeichnet, sind neben Methionin-Hydantoin (Hydantoin) auch z.B. Methioninamid (Met-amid), Methionin-Hydantoinsäureamid (Hyd-amid), Methionin-Hydantoinsäure (Hyd-säure) und Methionylmethionin (Met-Met), wobei 1 moleq Met-Met quantitativ betrachtet 2 moleq Methioninequivalenten entspricht. Analytisch bestimmt werden können diese einfach per HPLC oder als Summenparameter mit Schwefelspezifischer Titration z.B. mit Bromid/Bromat.
Die Konzentration der Methioninequivalente im Gemisch liegt typischerweise im Bereich zwischen 20 und 60 Gew.%, vorzugsweise bei ungefähr 40 Gew.%.
Der Entgeistungsschritt b) kann vorteilhaft bei Temperaturen von 80 bis 180°C, vorzugsweise von 100 bis 120°C und Drücken von 0 bis 1 barü durchgeführt werden, bevorzugt dergestalt, dass man die heiße Lösung aus der Verseifung in einen Stripper hinein entspannt ("flashing"). Dabei werden Ammoniak und Kohlendioxid praktisch vollständig entfernt.

Beide Schritte a) und b) können bei kontinuierlicher Verfahrensweise gleichzeitig, vorzugsweise in einer Verseifungskolonne, z.B. einer Siebboden-, Schlitzboden- oder Glockenbodenkolonne, oder aber getrennt nacheinander durchgeführt werden.

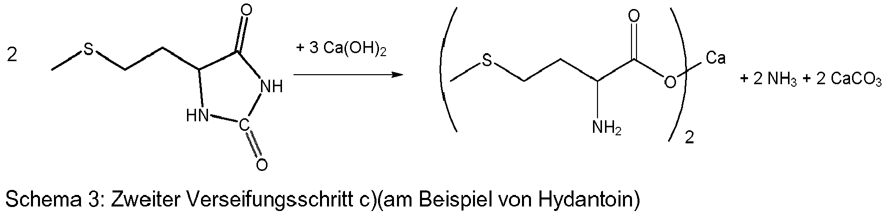

Der Hydrolyse-Schritt c) (Schema 3) kann vorteilhaft bei Temperaturen von 150 bis 190°C, vorzugsweise von 160 bis 185°C und Drücken von 5 bis 20 barü durchgeführt werden.

Dabei können im Hydrolyse-Schritt c) von 0,5 bis über 1,0 moleq Ca(OH)₂, vorzugsweise von 0,6 bis 0,8 moleq Ca(OH)₂ pro moleq Methioninequivalent verwendet werden. Dies gewährleistet wiederum hohe Umsätze bei relativ kurzen Verweilzeiten. Der Schritt c) kann bei kontinuierlicher Verfahrensweise am ökonomischsten in einem Plug-Flow-Reaktor durchgeführt werden, also einem Reaktor ohne Rückvermischung wie z.B. einem kontinuierlich betriebenen idealen Strömungsrohrreaktor.

Der nachfolgende Entgeistungschritt d) kann bei Temperaturen von 70 bis 100°C, vorzugsweise von 80 bis 95°C und Drücken von 0,5 bis 0,9 bara durchgeführt werden. Er wird vorzugsweise unter milden Bedingungen z.B. in einem Fallfilmverdampfer durchgeführt, um das Produkt möglichst rein und ohne thermische Umwandlungsprodukte zu erhalten.

Der nachfolgende Sprühgranulationsschritt e) kann bei Temperaturen von 50 bis 130°C, vorzugsweise von 55 bis 110°C und Drücken von 0,9 bis 1.2 bara durchgeführt werden Dabei eignen sich insbesondere Luft und Stickstoff als Gaskomponente, die z.B. über eine Zweistoffdüse zusammen mit der wässrigen Ausgangsmischung als Flüssigkomponente eingebracht wird. Die Sprühgranulation wird dabei vorzugsweise in einer Wirbelschicht durchgeführt, wahlweise nach dem Bottom-Spray oder Top-Sprayverfahren. Ein solches Verfahren ermöglicht die Bildung besonders gleichförmiger Teilchen mit relativ enger Korngrößenverteilung. Die im Austrag zu erzielende Korngröße ist dabei von der Keimbilanz in der Wirbelschichtanlage abhängig. Diese wird wesentlich vom Gleichgewicht von Keimbildung durch Abrieb oder nicht treffende Sprühtropfen und dem Granulataufbau bestimmt. Gezielt kann die Korngröße durch die Wahl der Trocknungs- und der Sprühparameter sowie durch den Einsatz eines Zerhackers eingestellt werden. Die so erzeugten Granulate können durch eine klassierende Einrichtung (z.B. Sichter, Unterlaufwehr) in angestrebter Partikelgröße aus dem Trocknungsraum ausgetragen werden. Die Partikelgröße des Produktes kann durch eine nachgeschaltete Siebung nach Wahl eingestellt werden. Das Überkorn der Siebung kann vorteilhafterweise über eine Mühle zerkleinert und gemeinsam mit dem z.B. mittels eines Zyklons abgetrennten Feinkorn zurückgeführt (z.B. pneumatisch oder mittels Förderschnecke). Dies hat den Zweck, dass ausreichend Keime für die Granulation im Prozessraum vorhanden sind und kein Produkt verloren geht.

Es kann weiterhin von Vorteil sein, dass vor Schritt e) eine zusätzliche Aufkonzentration des Rohproduktes aus Schritt d), vorzugsweise durch Eindampfen, erfolgt, insbesondere dann, wenn noch zuviel Wasser vorhanden ist, was die Sprühgranulation nur unnötig belasten würde. Diese Aufkonzentration kann auch simultan mit Schritt d) durchgeführt werden.

Im industriellen Maßstab ist es vorteilhaft, das Verfahren so durchzuführen, dass das in Schritt a) eingesetzte 5-(2-Methylmercaptoethyl)-hydantoin in Form eines 5-(2-Methylmercaptoethyl)-hydantoin enthaltenden Reaktionsgemisches eingesetzt wird, welches durch Umsetzung von Methylmercaptopropionaldehyd (MMP) mit Blausäure, Ammoniak und Kohlendioxid und/oder vom entsprechenden MMP-Cyanhydrin (MMP-CH) mit Ammoniak und Kohlendioxid erzeugt worden ist, also aus den üblichen Rohstoffen der industriellen Methioninsynthese. Dazu kann direkt eine bestehende Methioninanlage verwendet werden, aus deren "Hydantointeil" ein entsprechender Seitenstrang abgezweigt wird.
Dabei ist es von zusätzlichem Vorteil, wenn man aus dem eingesetzten Reaktionsgemisch restliches Ammoniak und Kohlendioxid aus der Hydantoinsynthese zuvor wenigstens teilweise durch Abdestillieren und/oder Abstrippen entfernt. Insbesondere die Abreicherung von Kohlendioxid erhöht den pH und erleichtert den anschließenden Hydrolyse-Schritt a) bzw. ermöglicht etwas geringere Ammoniakzufuhr in diesem Schritt.
Das dabei entfernte Ammoniak und Kohlendioxid gehen nicht verloren, sondern werden bevorzugt zur Erzeugung des 5-(2-Methylmercaptoethyl)-hydantoin enthaltenden Reaktionsgemisches wiederverwendet.

Ebenso kann auch das im Entgeistungsschritt b) abgetrennte Ammoniak und Kohlendioxid zur Erzeugung des 5-(2-Methylmercaptoethyl)-hydantoin enthaltenden Reaktionsgemisches wiederverwendet werden.
Desweiteren kann auch Ammoniak aus dem gemäß Schritt b) abgetrennten Gemisch aus Ammoniak und Kohlendioxid abgetrennt werden und zur Hydrolyse von 5-(2-Methylmercaptoethyl)-hydantoin gemäß Schritt a) wiederverwendet werden. Diese Gastrennung kann z.B. durch die üblichen Destillations- und Strippungsverfahren wahlweise kombiniert mit entsprechenden Wäschersystemen oder direkt durch ein "pressure swing"- System erfolgen.
Durch das erfindungsgemäße Verfahren gelingt es in relativ einfacher und kostengünstiger Weise aus industriell verfügbaren Rohstoffen direkt eine feste Calciummethioninatqualität mit einem Methionin-Gehalt im Endprodukt von >65 Gew-% herzustellen. Das Produkt weist gute Handhabungseigenschaften auf, wie z.B. hohe Rieselfähigkeit und geringe Verklumpungsneigung. Dabei bietet das organisch gebundene Calcium einen zusätzlichen Nutzen für die Tierernährung, da Calcium im Futtermittel erwünscht ist und daher nicht entfernt werden muss.

Das erfindungsgemäß hergestellte Calciummethioninat kann daher vorteilhaft direkt als Futtermitteladditiv in Tierfutter bzw. zur Herstellung von Tierfutter verwendet werden.

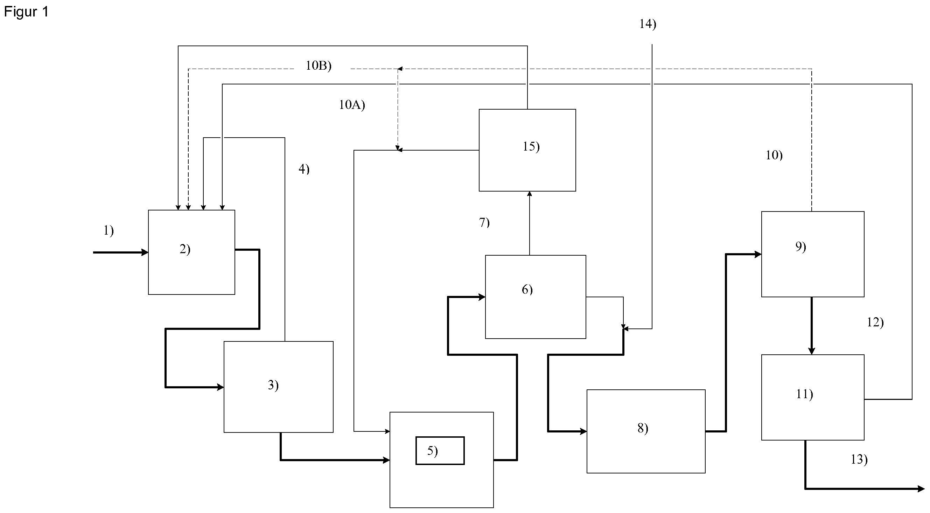

### Beschreibung des erfindungsgemäßen Verfahrens anhand eines Blockfließbildes (Figur 1)

Das Blockfließbild (Figur 1) zeigt die prinzipiellen Schritte und Ströme eines bevorzugten Verfahrens zur Herstellung von Ca(Met)₂ in einem industriellen Maßstab.

### Hydantoin-Synthese:

Hier wird durch Umsetzung von Methylmercaptopropionaldehyd (MMP) mit Blausäure, Ammoniak und Kohlendioxid und/oder vom entsprechenden MMP-Cyanhydrin (MMP-CH) mit Ammoniak und
Kohlendioxid ein 5-(2-Methylmercaptoethyl)-hydantoin enthaltendes Reaktionsgemisches erzeugt, also aus den üblichen Rohstoffen der industriellen Methioninsynthese. Dabei kann es sich um den Hydantointeil einer bestehenden Methioninanlage handeln, aus der ein entsprechender Seitenstrang abgezweigt wird.

**Entfernung von überschüssigem CO₂ und NH₃ (Flashing)** Hier wird das überschüssige CO₂ und Ammoniak aus dem Hydantoin-Strom abgetrennt, um die Neutralisierung von zugegebenem Ammoniak zu verhindern.

### a) Erster Verseifungsschritt

Die teilweise Verseifung von Hydantoin-Verbindungen (neben Methionin-Hydantoin z.B. Methioninamid, Methionin-Hydantoinsäureamid und Methionin-Hydantoinsäure) mit Hilfe von Ammoniak findet hier unter hohem Druck und höherer Temperatur statt. Es kann beispielsweise ein kontinuierlich betriebener idealer Rührkesselreaktor, also ein Reaktor mit totaler Rückvermischung (= Continuous Stirred-Tank Reactor/CSTR-Reaktor) eingesetzt werden oder eine Verseifungskolonne, z.B. der weiter oben genannten Art (vgl. Textabschnitt zwischen Schema 2 und 3).

### b) Entgeistungsschritt (Abtrennung von überschüssigem NH₃ und CO₂)

Ammoniak muss in dieser Stufe entfernt werden, um ihn z.B. im ersten Verseifungsschritt wieder einsetzen zu können. Das freigesetzte CO₂ aus den ersten Verseifungsschritt muss hier vor der zweiten Verseifung entfernt werden, andernfalls würde es chemisch mit Ca(OH)₂ neutralisiert in Form von Calciumcarbonat und dadurch einen erhöhten Calciumoxidverbrauch hervorrufen bzw. einen erhöhten Anteil von Calciumcarbonat im Produkt. Dieser Schritt kann vorteilhaft in einer Strippkolonne ähnlich einer Verseifungskolonne aus dem Methioninprozess stattfinden. Die gemeinsam abgetrennten Gase NH₃ und CO₂ können als Gemisch direkt in den Hydantointeil einer damit verbundenen Methionin-Anlage zurückgeführt werden.

### Optionale Trennung von NH₃ und CO₂

Für den Fall, dass der in b) zurückgewonnene Ammoniak zusammen mit Wasseranteilen in den ersten Verseifungsschritt zurückgeführt werden soll, ist das CO₂ aus dem ersten Verseifungsschritt hier nicht erwünscht und muss deshalb minimiert werden.

### c) Zweiter Verseifungsschritt

Hier werden nach Zugabe von Ca(OH)₂, welches auch einfach durch Zugabe von Calciumoxid in das wässrige System in situ erzeugt werden kann, die restlichen Methionin-Hydantoin-Verbindungen verseift zu Methionin und einen Rest von Calciumcarbonat, welcher im Produkt verbleiben kann.

### d) Entgeistungsschritt (Abtrennung von NH₃ und Evaporation von H₂O)

Ammoniak muss in dieser Stufe entfernt werden, um ihn z.B. im ersten Verseifungsschritt wieder einsetzen zu können (Option A) oder wahlweise in der Hydantoin-Synthese einer angeschlossenen Methionin-Anlage (Option B).

### Optional: Eindampfen/Aufkonzentration (nicht gezeigt in Figur 1)

Hier wird zusätzlich noch überschüssiges Wasser vor der Sprühgranulation entfernt.

### e) Sprühgranulation

In diesem Schritt wird das gewünschte Endprodukt nämlich granuliertes Ca(Met)₂ mit Resten Calciumcarbonat durch Sprühgranulation des entgeisteten Hydrolysats II erzeugt.

### Beispiele

### Zusammensetzung der eingesetzten technischen Methionin-Hydantoin- Lösungen (vgl. ergänzende Angaben in Tabelle)

Met-amid: 1,5 Gew.%
Hyd-amid: 8 Gew.%
Hyd-säure: 1,5 Gew.%
Hydantoin: 24 Gew.%
Alle Methioninvorstufen inklusive der vorstehend genannten Komponenten werden in Summe in diesem Zusammenhang auch als "Hyd" bezeichnet.

### Aufkonzentrieren der Methionin-Hydantoinlösungen

Eine technische Hydantoin-Lösung aus einer Methioninanlage wurde in einem Rotationsverdampfer auf ca. die Hälfte ihres Originalvolumens eingedampft bei 70 °C Badtemperatur, 150-200 mbar Druck innerhalb von: 2,5 - 3 h.

Typische Zusammensetzung:
Met-amid: 2 Gew.%
Hyd-amid: 17 Gew.%
Hyd-säure: 5 Gew.%
Hydantoin: 49 Gew.%

### Strippen der Lösungen

Die vorgeheizte Lösung (ca.70 °C) wurde durch eine mit Raschigringen gefüllte Strippkolonne geführt (105 °C Manteltemperatur, Dosierraten: 4 g/min Dampf, 250 g/h Lösung) und damit gasförmige Anteile wie Ammoniak und CO₂ abgereichert.

### Verseifung im Roth-Autoklaven mit NH₃ zur Erzeugung des Hydrolysats I

### - Verseifung mit wäßrigem Ammoniak

Verflüssigte Hydantoin-Lösung (mit ca.70 °C) wurde in einen Roth-Autoklaven überführt. Wässriger Ammoniak (ca. 33 Gew.%) wurde zugegeben und der Autoklav sofort verschlossen.

Der Autoklav wurde dann unter Rühren mit einem Autoklavenofen auf die gewünschte Temperatur im Bereich von 140 -180°C erhitzt und bei dieser für eine gewünschte Zeit gehalten (vgl. Tabelle 1).

Nach der gegebenen Zeit wurde der Autoklav für 20 min in einem Wasserbad abgekühlt bis eine Innentemperatur von 18 °C erreicht war.

### - Verseifung mit Ammoniakgas

Eine verflüssigte homogene Hydantoin-Lösung (mit ca.70 °C) wurde in einen Roth-Autoklaven überführt.

Bei einer Innentemperatur von 40 °C wurde eine gewünschte Menge Ammoniakgas aus einer Ammoniakbombe (6 bar) für ca. 1 Stunde in den Autoklaven geleitet.

Danach wurde die Ammoniakbombe entfernt, der Autoklav verschlossen und unter Rühren bis zur gewünschten Temperatur (vgl. Tabelle 1) in einem Autoklavenofen aufgeheizt. Nach der vorgegebenen Zeit wurde der Autoklav für 20 min in einem Wasserbad abgekühlt bis eine Innentemperatur von 18 °C erreicht war.

Die Menge an Ammoniak, welche zugegeben worden war, wurde bestimmt durch Wiegen der Reaktionsmischung nach der Reaktion. Ein Vergleich der so bestimmten Ammoniakmenge im verschlossenen Autoklav und nach dessen Öffnung zeigte praktisch keine Differenzen. Deshalb muss davon ausgegangen werden, dass das Öffnen des Autoklaven nicht zu signifikanten Verlusten an Ammoniak geführt hat und die eingewogene Menge Ammoniak auch während der gewählten Reaktionszeit zur Verfügung gestanden hat.

Es wurde eine Probe genommen und per HPLC analysiert und so der Umsatz der Methioninequivalente bzw. des Methionin-Hydantoins (Hyd) bestimmt.

### Bestimmung des CO₂-Gehalts in der mit NH₃ vorverseiften Lösung (Hydrolysat I)

Es wurden 25 g der entsprechenden Lösung auf 100 mL Gesamtvolumen verdünnt. Es wurden 15 mL einer 10% Ba(OH)₂-Lösung zugefügt, was zum Ausfallen eines Niederschlags an gebildetem Bariumcarbonat führte. Der Niederschlag wurde filtriert und zusätzliche Bariumhydroxidlösung zugefügt, um sicherzustellen, dass kein weiterer Niederschlag erfolgen würde. Der gesamte Niederschlag an so gebildetem Bariumcarbonat wurde getrocknet und gewogen und daraus der ursprüngliche CO₂-Gehalt in der Lösung bzw. der Anteil an freigesetztem CO₂ berechnet.

**Tabelle 1: Ergebnisse der Verseifungen mit NH₃**

| **Beispiel** | **Temperatur** | **Zeit** | **NH₃ : Hyd (Verseifungsverhältnis)** | **Konzentration Hyd** | **Umsatz** | **Wieder-findungsrate** | **CO₂-Entfernungsrate** |
|---|---|---|---|---|---|---|---|
| Nr. | [°C] | [h] | [ mol/mol] | [mol/kg] | [Mol%] | [Mol%] | [Mol%] |
| 1 | 160 | 0,25 | 10 | 0,9 | 74% | 85% | 66% |
| 2 | 160 | 0,5 | 10 | 0,9 | 88% | 83% | 80% |
| 3 | 160 | 1 | 10 | 0,9 | 94% | 79% | 85% |
| 4 | 160 | 1,5 | 10 | 0,9 | 98% | 75% | 85% |
| 5 | 140 | 0,25 | 8 | 1,3 | 40% | 90% | 35% |
| 6 | 160 | 0,25 | 8 | 1,3 | 72% | 80% | 60% |
| 7 | 170 | 0,25 | 8 | 1,3 | 80% | 74% | 70% |
| 8 | 180 | 0,25 | 8 | 1,3 | 88% | 70% | 81% |
| 9 | 160 | 0,25 | 20 | 1,4 | 68% | n.b. | n.b. |
| 10 | 160 | 0,5 | 2,0 | 1,4 | 75% | n.b. | n.b. |
| 11 | 160 | 0,25 | 6,4 | 1,4 | 88% | n.b. | n.b. |
| 12 | 160 | 0,5 | 6,4 | 1,4 | 90% | n.b. | n.b. |
| 13 | 170 | 0,25 | 2,0 | 1,4 | 72% | n.b. | n.b. |
| 14 | 170 | 0,25 | 6,4 | 1,4 | 85% | n.b. | n.b. |
| 15 | 180 | 0,25 | 2,0 | 1,4 | 80% | n.b. | n.b. |
| 16 | 180 | 0,25 | 6,4 | 1,4 | 90% | n.b. | n.b. |

| | | | | | | | |
|---|---|---|---|---|---|---|---|
| n.b. = nicht bestimmt | | | | | | | |

Höhere Temperaturen führen zu höheren Umsätzen wie z.B. die Temperaturvariation der Beispiele 5 bis 8 zeigt. Auch bei niedrigeren Temperaturen als 160°C, z.B. bei 140°C können aber noch akzeptable Hydrolyseergebnisse erzielt werden, insbesondere wenn man dafür die Reaktionszeit verlängert. Der Anstieg des Umsatzes bei gegebener Temperatur mit der Reaktionszeit ist z.B. an den Beispielen 1 bis 4 gut erkennbar. Auch ein höheres Verseifungsverhältnis NH₃ : Hyd vergrößert die Umsatzrate, wie der Vergleich der Beispiele 9, 6 und 1 oder 9 und 10, 11 und 12, 13 und 14 oder 15 und 16 zeigt.

Als Vorbereitung für den nächsten Schritt wurden die gasförmigen Komponenten aus dem Hydrolysat I durch Eindampfen in einem Rotationsverdampfer (3 h, 70 °C Badtemperatur, 200 mbar) entfernt und so ein entgeistetes Hydrolysat I erzeugt.

Alternativ wurden die gasförmigen Komponenten durch Eindampfen der Lösung über eine Strippkolonne gefüllt mit Raschigringen entfernt (105 °C Temperatur im Heizmantel, 4 g/min Dampf, 250 g/h Lösung).

### Verseifung im Roth-Autoklaven mit Ca(OH)₂ zur Erzeugung des Hydrolysats II

Eine derart mit Hilfe von Ammoniak vorverseifte und entgeistete Met-Hydantoin-Lösung (entgeistetes Hydrolysat I) wurde gemischt mit Ca(OH)₂ und (falls erforderlich) Wasser, in einem Roth-Autoklaven.

### Zusammensetzung der Startlösung (Pro 100g)

Met-Amid: 6,54 Gew.% entsprechend 6,54g (44,16 mmol)
Met: 8,01 Gew.% entsprechend 8,01g (53,72 mmol)
Hyd-Amid: 1,80 Gew.% entsprechend 1,80g (9,38 mmol)
Hyd-Säure: 3,53 Gew.% entsprechend 3,53g (18,48 mmol)
Hydantoin: 4,78 Gew.% entsprechend 4,78g (27,47 mmol)
Met-Met: 1,74 Gew.% entsprechend 1,74g (6,21 mmol)

### Summe der Methioninequivalente (ME): 165,63 mmol (entspricht 100 Mol%)

Der Autoklav wurde verschlossen und in einem Autoklav-Heizofen aufgeheizt. Es wurde ca. alle 10 Minuten ein Ventil vorsichtig geöffnet, um 2-4 bar Druck abzulassen und damit dem gebildeten Ammoniak Gelegenheit zu geben, das System zu verlassen.

**Tabelle 2: Ergebnisse der Verseifungsversuche mit Ca(OH)₂**

| **Beispiel** | **Temperatur** | **Zeit** | **Ca²⁺: Hyd (Verseifungsverhältnis)** | **Konzentration ME** | **Umsatz** | **Wiederfindungsrate** |
|---|---|---|---|---|---|---|
| Nr. | [°C] | [h] | [ mol/mol] | [mol/kg] | [Mol%] | [Mol%] |
| 17 | 160 | 1 | 1,00 | 1,6 | 100% | 70% |
| 18 | 160 | 1 | 0,75 | 1,6 | 88% | 69% |
| 19 | 150 | 1 | 0,75 | 1,6 | 77% | 75% |
| 20 | 170 | 1 | 0,75 | 1,6 | 90% | 61% |
| 21 | 170 | 0,5 | 0,75 | 1,6 | 88% | 69% |
| 22 | 160 | 1 | 0,50 | 1,6 | 80% | 54% |
| 23 | 180 | 0,5 | 0,75 | 1,6 | 90% | 60% |
| 24 | 180 | 0,5 | 0,50 | 1,6 | 75% | 55% |
| 25 | 180 | 0,5 | 0,50 | 1,6 | 72% | 58% |
| 26 | 160 | 1 | 0,50 | 1,6 | 70% | 70% |
| 27 | 160 | 1 | 1,00 | 2,3 | 59% | 45% |
| 28 | 180 | 0,5 | 1,00 | 2,2 | 62% | 30% |
| 29 | 160 | 0,5 | 1,00 | 2,9 | 71% | 45% |
| 30 | 160 | 1 | 1,00 | 2,6 | 73% | 32% |

Höhere Temperaturen bei sonst gleichen Bedingungen führen zu höheren Umsätzen wie z.B. die Temperaturvariation der Beispiele 19 und 20, 21 und 23 zeigt.

Auch bei niedrigeren Temperaturen als 160°C, z.B. bei 150°C können aber noch akzeptable Hydrolyseergebnisse erzielt werden (Beispiel 19), insbesondere wenn man dafür die Reaktionszeit verlängert.

Der Anstieg des Umsatzes mit der Reaktionszeit bei gegebener Temperatur ist z.B. an den Beispielen 21 und 20 (bei sonst gleichen Bedingungen) sowie an den Beispielen 29 und 17 (hier mit zusätzlicher Veränderung der Hydantoin-Konzentration) gut erkennbar.

Auch ein höheres Verseifungsverhältnis Ca²⁺: Hyd vergrößert die Umsatzrate, wie der Vergleich der Beispiele 17,18 und 22 oder 23 und 24 zeigt.

### Wirbelschicht-Sprühgranulation des Hydrolysats II

Die Beispiele wurden in einer Laborwirbelschichtsprühgranulations-Apparatur T200 (umgebauter Aeromatic T2 der Firma Formpak (Pty) Ltd./Süd Afrika mit zylindrischem Trocknerteil DN 200, Siebboden mit Metall-Siebgewebe mit der Maschenweite von ca. 90 µm, mit seitlich eingesteckter Düse und Sprührichtung von unten nach oben) in batch-Fahrweise durchgeführt. Dabei wurden die folgenden Versuchsparameter eingestellt.

Als Startfüllung wurde D,L- Methionin, feed grade in die Sprühgranulationsapparatur eingefüllt.

Handhabung der Suspension:
Portionen zu je 0,8 L wurden in einer Braun K3000-Küchenmaschine mit Pürieraufsatz vordispergiert und in ein IKB magic LAB-Labormaschine (mit MKO-Einheit) eingefüllt. Von dort wurde die Suspension dann mit einer Schlauchpumpe zur Düse gefördert und mittels Druckluft (2,3 barü) in die Wirbelschicht eingesprüht.

### Eingestellte Parameter bei der Wirbelschicht-Sprühgranulation:

T Zuluft = 88 - 90°C
T Wirbelbett = 57 - 79°C
T Abluft = 55 - 79°C
Sprührate = 2,4 kg/h Hydrolysat II
Düsendruck (Zweistoffdüse) = ca.1 bara
Volumenstrom Trocknungsluft = 118 - 132 m³/h.
Anlagendruck oberhalb des Siebbodens = ca.10 mbar unterhalb Atmosphärendruck

Es wurde dabei ein Calciummethioninat-Sprühgranulatmusterprodukt erzeugt, das gut rieselfähig und nicht klumpend war, ebenso auch nicht hygroskopisch. Das Produkt wies aufgrund seiner hohen Rieselfähigkeit und geringen Verklumpungsneigung gute Handhabungseigenschaften auf.

### Bezugszeichenliste zu Figur 1

- 1): Rohstoffe
- 2): Hydantoin-Synthese (Hydantointeil einer Methioninanlage)
- 3): Abtrennung von überschüssigem CO₂, NH₃
- 4): Stoffstrom, abgetrenntes NH₃ und CO₂
- 5): a) Erster Verseifungsschritt (mittels NH₃)
- 6): b) Entgeistungsschritt, Abtrennung von NH₃ und CO₂
- 7): Stoffstrom, abgetrenntes NH₃ und CO₂, H₂O
- 8): c) Zweiter Verseifungsschritt (mittels Ca(OH)₂)
- 9): d) Entgeistungsschritt, Abtrennung von NH₃ und Evaporation von H₂O
- 10): Stoffstrom, abgetrenntes NH₃ und H₂O
- 10A): Option A
- 10B): Option B
- 11): e) Sprühgranulation
- 12): Stoffstrom abgetrenntes H₂O und NH₃
- 13): Granuliertes Produkt CaMet₂
- 14): CaO-Slurry
- 15): Optionale Trennung von NH₃ und CO₂

## Patentansprüche

1. Verfahren zur Herstellung von Calciummethioninat, **gekennzeichnet durch** folgende Schritte:
a) Hydrolyse von 5-(2-Methylmercaptoethyl)-hydantoin mit Ammoniak und Wasser zu einem Ammoniummethioninat und Kohlendioxid enthaltenden Hydrolysat I,
b) Erzeugung eines entgeisteten Hydrolysats I durch wenigstens teilweise Abtrennung von vorhandenem Ammoniak und Kohlendioxid aus dem Hydrolysat I, vorzugsweise durch Abdestillieren und/oder Abstrippen,
c) Hydrolyse des entgeisteten Hydrolysats I aus b) mit CaO und/oder Ca(OH)₂ und Wasser zu einem Calciummethioninat und Ammoniak enthaltenden Hydrolysat II,
d) optional Erzeugung eines Calciummethioninat enthaltenden Rohprodukts durch Abtrennung von vorhandenem Ammoniak aus dem Hydrolysat II aus c), vorzugsweise durch Abdestillieren und/oder Abstrippen, und
e) Erzeugung von festem Calciummethioninat durch Sprühgranulation des Rohproduktes aus d) oder gegebenenfalls durch direkte Sprühgranulation des Calciummethioninat und Ammoniak enthaltenden Hydrolysats II aus c).

2. Verfahren gemäß Anspruch 1 **dadurch gekennzeichnet, dass** Hydrolyse-Schritt a) bei Temperaturen von 140 bis 200°C, vorzugsweise von 155 bis 185°C und besonders bevorzugt von 160 bis 180°C und Drücken von 10 bis 30 barü durchgeführt wird.

3. Verfahren gemäß Anspruch 1 oder 2 **dadurch gekennzeichnet, dass** im Hydrolyse-Schritt a) von 1 bis 20 moleq NH₃, vorzugsweise von 2 bis 10 moleq NH₃ pro moleq Methioninequivalent verwendet werden.

4. Verfahren gemäß einem der Ansprüche 1 bis 3, **dadurch gekennzeichnet, dass** Abtrennungs-Schritt b) bei Temperaturen von 80 bis 180°C, vorzugsweise von 100 bis 120°C und Drücken von 0 bis 1 barü durchgeführt wird.

5. Verfahren gemäß einem der Ansprüche 1 bis 4, **dadurch gekennzeichnet, dass** Hydrolyse-Schritt c) bei Temperaturen von 150 bis 190°C, vorzugsweise von 160 bis 185°C und Drücken von 5 bis 20 barü durchgeführt wird.

6. Verfahren gemäß einem der Ansprüche 1 bis 5 **dadurch gekennzeichnet, dass** im Hydrolyse-Schritt c) von 0,5 bis 1,0 moleq Ca(OH)₂, vorzugsweise von 0,6 bis 0,8 moleq Ca(OH)₂ pro moleq Methioninequivalente verwendet werden.

7. Verfahren gemäß einem der Ansprüche 1 bis 6, **dadurch gekennzeichnet, dass** Abtrennungs-Schritt d) bei Temperaturen von 70 bis 100°C, vorzugsweise von 80 bis 95°C und Drücken von 0,5 bis 0,9 bara durchgeführt wird.

8. Verfahren gemäß einem der Ansprüche 1 bis 7, **dadurch gekennzeichnet, dass** Sprühgranulationsschritt e. bei Temperaturen von 50 bis 130°C, vorzugsweise von 55 bis 110°C und Drücken von 0,9 bis 1,2 bara durchgeführt wird.

9. Verfahren gemäß einem der Ansprüche 1 bis 8, **dadurch gekennzeichnet, dass** vor Schritt e) eine zusätzliche Aufkonzentration des Rohproduktes aus Schritt d), vorzugsweise durch Eindampfen, erfolgt.

10. Verfahren gemäß einem der Ansprüche 1 bis 8, **dadurch gekennzeichnet, dass** das in Schritt a) eingesetzte 5-(2-Methylmercaptoethyl)-hydantoin in Form eines 5-(2-Methylmercaptoethyl)-hydantoin enthaltenden Reaktionsgemisches eingesetzt wird, welches durch Umsetzung von Methylmercaptopropionaldehyd (MMP) mit Blausäure, Ammoniak und Kohlendioxid und/oder vom entsprechenden MMP-Cyanhydrin (MMP-CH) mit Ammoniak und Kohlendioxid erzeugt worden ist.

11. Verfahren gemäß Anspruch 10 **dadurch gekennzeichnet, dass** aus dem eingesetzten Reaktionsgemisch restliches Ammoniak und Kohlendioxid zuvor wenigstens teilweise durch Abdestillieren und/oder Abstrippen entfernt worden ist.

12. Verfahren gemäß Anspruch 11, **dadurch gekennzeichnet, dass** entferntes Ammoniak und Kohlendioxid zur Erzeugung des 5-(2-Methylmercaptoethyl)-hydantoin enthaltenden Reaktionsgemisches gemäß Anspruch 10 wiederverwendet wird.

13. Verfahren gemäß Anspruch 10 **dadurch gekennzeichnet, dass** gemäß Anspruch 1 Schritt b) abgetrenntes Ammoniak und Kohlendioxid zur Erzeugung des 5-(2-Methylmercaptoethyl)-hydantoin enthaltenden Reaktionsgemisches gemäß Anspruch 10 wiederverwendet wird.

14. Verfahren gemäß einem der Ansprüche 1 bis 13, **dadurch gekennzeichnet, dass** Ammoniak aus dem gemäß Anspruch 1 Schritt b) abgetrennten Gemisch aus Ammoniak und Kohlendioxid abgetrennt wird und zur Hydrolyse von 5-(2-Methylmercaptoethyl)-hydantoin gemäß Anspruch 1 Schritt a) wiederverwendet wird.

15. Verwendung des gemäß einem der Ansprüche 1 bis 14 hergestellten Calciummethioninats als Futtermitteladditiv in Tierfutter.

16. Verwendung des gemäß einem der Ansprüche 1 bis 14 hergestellten Calciummethioninats als Futtermitteladditiv zur Herstellung von Tierfutter.
